Europäisches Patentamt

European Patent Office

Office européen des brevets

(11) Publication number: **0 269 979**
**A2**

## (12) EUROPEAN PATENT APPLICATION

(21) Application number: 87117321.7

(51) Int. Cl.⁴: **C12Q 1/42** , G01N 33/52

(22) Date of filing: 24.11.87

(30) Priority: 28.11.86 JP 281831/86

(43) Date of publication of application:
08.06.88 Bulletin 88/23

(84) Designated Contracting States:
DE FR GB IT SE

(71) Applicant: KONICA CORPORATION
No. 26-2, Nishi-shinjuku 1-chome
Shinjuku-ku Tokyo(JP)

(72) Inventor: Koyama, Mikio
c/o Konica Corp.1,Sakuramachi
Hino-Shi Tokyo(JP)

(74) Representative: Henkel, Feiler, Hänzel &
Partner
Möhlstrasse 37
D-8000 München 80(DE)

(54) Analytical element for measuring alkaline phosphatase.

(57) There is disclosed a multi-layer analytical element for measuring alkaline phosphatase in a liquid sample, which comprises a substrate for measuring activity of alkaline phosphatase and a buffering agent having a pKa value of about 8 or higher contained in layers separate from each other, characterized in that the substrate is a phosphoric acid ester of a non-self-developable compound or its salt, and the non-self-developable compound formed by the action of alkaline phosphatase is a compound capable of forming a dye through condensation of two molecules in the presence of an oxidizing agent.

## Analytical element for measuring alkaline phosphatase

## BACKGROUND OF THE INVENTION

This invention relates to a dry system multi-layer analytical element for analysis of a biological fluid, for example, blood concerning alkaline phosphatase.

In clinical test, it is very important to measure activity of alkaline phosphatase in human body fluid. More specifically, measurement of activity of alkaline phosphatase (hereinafter abbreviated as ALP) in body fluid has been done for diagnosis of liver diseases such as acute hepatitis, hepatocirrhosis, hepatoma, etc., bone diseases including osteoma, and biliary tract diseases, etc.

For the method for measuring ALP activity, various methods have been investigated since the method of H.D. Kay [Journal of Biological Chemistry, Vol. 89, p. 235 (1930)]. Among them, remarkable effect was brought about to simplification of the measuring method by use of arylphosphates developed by E.J. King et al. [The Biochemical Journal, Vol. 33, p. 1185 (1939)], and further p-nitrophenylphosphoric acid having self-developability as the substrate.

The above measuring method is being established as the IFCC method (Clinica Chemical Acta (1983) 339F - 367F), and it has been known that the optimum pH for the reaction is around 10, existence of an inorganic salt, particularly magnesium ion as the activator has been made clear, and disodium p-nitrophenylphosphate is used as the substrate including utilization of a preferable co-existing alcohol as the receptor for phosphoric acid formed.

However, disodium p-nitrophenylphosphate is poor in stability and its solution can be stored at 20 to 26 °C limitedly for 8 hours, whereby there is inconvenience that the substrate solution must be prepared during measurement. Further, disodium p-nitrophenylphosphate is also unstable at a freezer temperature, as shown by E. Anador et al., Journal of American Medicinal Association, Vol. 184, p. 953 (1963), and therefore, not only purity assay of the reagent is performed before measurement but also special care is required from after preparation of the reagent stock to measurement.

As a method for ameliorating instability of the above p-nitrophenylphosphoric acid, an organic amine salt of p-nitrophenylphosphoric acid is proposed in Japanese Patent Publication No. 34872/1970. By use of this salt, stability of the substrate itself was improved, whereby it became possible to assemble the substrate in a kit for measurement of ALP.

On the other hand, in the region of clinical test, there is a strong demand to have simple operability and rapid measurement from those concerned. As the direction to respond to such demand, there have been recently developed dry system analytical methods, particularly multi-layer analytical elements which are particularly simple in handling and also good in precision, and further improvements thereof are under progress.

The above multi-layer analytical element is disclosed in U.S. Patent No. 4,555,484. According to the above patent specification, the element has a first reagent zone and a second reagent zone on a support, the first reagent zone containing a substrate of ALP and the second reagent zone containing a buffering agent which is an alkali metal salt of ammonium salt having a pKa value of about 9 to about 11.5. Further, in Japanese Provisional Patent Publication No. 237999/1985, it is disclosed that the above substrate is contained in fine powdery state, and said substrate is contained in the spreading layer. Also, in Japanese Provisional Patent Publication No. 110058/1986, it is disclosed that the element comprises a buffering agent layer containing one or more compounds functioning as the phosphoric acid receptor and the buffering agent and a spreading layer containing a substrate exhibiting self-developability.

Although these were proposed with particular attention on storage stability, they cannot be said to have sufficient stability.

Since the above substrate releases, by accepting an action of ALP, a developable compound due to hydrolysis, sensitivity of measurement depends upon molecular adsorption coefficient of the developable compound. While its stability is stable as compared with sodium p-nitrophenylphosphate, it cannot be said to have sufficient stability in the various use conditions and preservation conditions, and it has been known that non-enzymatic alkaline hydrolysis occurs. It has been known that not only this non-enzymatic hydrolysis increases a blank value (so-called fog value) at measurements but also the phosphoric acid formed remarkably inhibits an activity of ALP.

## SUMMARY OF THE INVENTION

An object of the present invention is to provide an analytical element for measuring ALP excellent in sensitivity and stability.

To outline the present invention, the present invention is an invention concerning an analytical element for measuring ALP, which is·a multi-layer analytical element for measuring alkaline phosphatase in a liquid sample, comprising a substrate for measuring activity of alkaline phosphatase and a buffering agent having a pKa value of about 8 or higher contained in layers separate from each other, characterized in that said substrate is a phosphoric acid ester of a non-self-developable compound or its salt, and the non-self-developable compound formed by the action of alkaline phosphatase is a compound capable of forming a dye through condensation of two molecules in the presence of an oxidizing agent.

The present inventors have investigated about various substrates for various ALP's and consequently found that a multi-layer analytical element for measuring ALP activity with excellent storage stability and high sensitivity can be obtained by use of a multi-layer analytical element containing a non-developable phosphoric acid ester, such as a substituted or unsubstituted indolyl phosphate or its salt and at least one buffering agent which buffers pH at around about 9.5 to about 12 and has a pKa value of about 8 or higher in layers separate from each other, to accomplish the present invention.

## DESCRIPTION OF THE PREFERRED EMBODIMENTS

In the following, the present invention will be described in more detail.

The substrate to be used in the present invention is a non-developable phosphoric acid ester. The term "non-developable" as mentioned in the present invention means that the substrate is subject to the action of ALP to be hydrolyzed, and the compound formed itself has no absorption at the visible region, and the above compound becomes a compound having absorption at the visible region through oxidative condensation of two molecules in the presence of an appropriate oxidizing agent.

Such substrates are described in J.P. Horwitz et al., Journal of Medicinal Chemistry, Vol. 9, p. 447 (1966), which are generally used as the substrates for enzymatic dyeing methods for the study of histological chemistry.

Examples of the substrate to be used in the present invention may include substituted or unsubstituted indolyl phosphate and salts thereof. Specific representative examples of substituted or unsubstituted indolyl phosphate and salts thereof may include, 3-indolyl phosphate and salts thereof, 5-bromo-3-indolyl phosphate and salts thereof, 5-bromo-4-chloro-3-indolyl phosphate, 5-bromo-4-iodo-3-indolyl phosphate and salts thereof, etc. The cation of the salt of the substituted or unsubstituted indolyl phosphate of the present invention may be selected from alkali metal ions such as sodium ion, potassium ion, and organic amines such as toluidine, tris(hydroxymethyl)aminomethane, 2-amino-2-methyl-1,3-propanediol, 2-amino-2-ethyl-1,3-propanediol, bis(cyclohexyl)amine, 2-(ethylamino)ethanol, N-methylglucamine, 2-(methylamino)ethanol, 2-(dimethylamino)ethanol, 2-(isopropylamino)ethanol and 2-amino-2-methyl-propanol. Preferably, organic amine salts may be selected.

The substrate for ALP of the present invention is subject to hydrolysis by the action of ALP in the presence of a buffering agent which buffers pH at around about 9.5 to about 12 and has a pKa value of about 8 or more to form phosphoric acid and a non-self-developable compound, for example, substituted or unsubstituted indoxyl. Said non-self-developable compound, for example, an indoxyl derivative has itself no absorption at the visible region. The above derivative can be oxidatively condensed with two molecules in the presence of an appropriate oxidizing agent to form a dye having absorption at the visible region.

The substrate of the present invention may be contained in any of the reagent layer, the porous spreading layer, etc., provided that it is not the same layer as the layer containing the buffering agent. For example, it can be incorporated according to the known method such as dissolution, impregnation, dispersion, etc. When it is contained in the porous spreading layer, the method should be suitably selected depending on the material forming the porous spreading layer.

As the porous spreading layer, there may be included fibrous spreading layer comprising separate fibers and a reactive polymer as disclosed in Japanese Provisional Patent Publication No. 197466/1982, non-fibrous porous medium layer, the so-called brush polymer layer as disclosed in Japanese Patent Publication No. 21677/1978, particulate bound product having particles bound directly or through a low molecular compound as disclosed in Japanese Provisional Patent Publications No. 101760/1982 and No. 101761/1982, particulate structure comprising particles and adhesive fine particles as disclosed in Japanese Provisional Patent Publication No. 90859/1980 and fabric subjected to hydrophilic treatment as disclosed in Japanese Provisional Patent Publication No. 164356/1980, etc.

The substrate of the present invention may be preferably contained in the porous spreading layer. In that case, as described in Japanese Provisional Patent Publication No. 237999/1985, the state of containing the substrate in fine powder is the best state for both stability and sensitivity. Further, the substrate of the present invention can be contained in the reagent layer. In this case, the substrate of the present invention should be preferably contained in a layer upper than the layer containing the buffering agent. More specifically, it is preferably contained as dispersed in the form of fine powder in a hydrophilic binder soluble in an organic solvent which does not dissolve the substrate of the present invention. The binder may be, for example, preferably polyvinyl pyrrolidone, a copolymer (vinyl pyrrolidone-vinyl acetate), methyl cellulose, ethyl cellulose, etc.

The non-developable compound formed from the substrate of the present invention by the action of ALP, for example, indoxyl or a derivative thereof, forms a dye through condensation of two molecules in the presence of an oxidizing agent. As the oxidizing agent, those generally known in the art such as metaperiodates, metabisulfites, persulfates and oxygen, etc. may be employed. Oxygen is preferred. Since oxygen is supplied through the porous spreading layer, the substrate of the present invention is preferably contained in the porous spreading layer. When another oxidizing agent is used, it may be contained in any layer, but preferably in the same layer as the layer containing the buffering agent.

The buffering agent may be one which buffers pH at around about 9.5 to about 12 and does not interfere with the activity of ALP. For example, there may be included carbonates, borates, those known as the Good's buffering agent, diethyanolamine, 2-amino-2-methyl-propanol, tris (hydroxymethyl)-aminomethane, diethylamine, 2-(methylamino)ethanol, 2-(isopropylamino)ethanol, and other known buffering agents. For example, reference can be made to McComb et al., Clinical Chemistry, Vol. 18, No. 2, pp. 97 to 104 (1972).

Although there exist those functioning as the phosphoric acid receptor in the above buffering agent, they are compounds having functional groups (hydroxyl group, etc.) more rapid in reaction with the phosphoric acid formed from the substrate by the action of ALP than the reaction between phosphoric acid and water (hydrolysis) and will not lower the enzymatic activity of ALP, because the phosphoric acid formed is an inhibitor against ALP. A phosphoric acid receptor may be assembled within the element, and an amino alcohol is preferred. Those generally accepted to be effective may include diethanolamine, 2-amino-2-methyl-1,3-propanediol, tris(hydroxymethyl)aminomethane, etc. Further, an amino alcohol with a pKa value of about 8 or more is effective, and it can be selected as desired from the compounds as described in the report of McComb et al. *supra*, such as 2-aminoethanol, 2-(ethylamino)ethanol, 2-(methylamino)ethanol, 2-(dimethylamino)ethanol, 2-(isopropylamino)ethanol, 2-amino-2-methylpropanol, 3-aminopropanol, DL-2-amino-propanol, hydroxyproline, N-methylglucamine, etc.

Since these phosphoric acid receptors can be also used as the buffering agent, they can be also used simultaneously as the buffering agent without addition of other buffering agents, if desired. The phosphoric acid receptor, when contained as other compound than the buffering agent, is preferably contained in the buffering agent layer.

The buffering agent and the substrate can be also contained in a layer nearer to the support than to the porous spreading layer. In this case, when the non-self-developable compound formed from the substrate is condensed with oxygen, the substrate of the present invention is preferably contained in a layer nearer to the porous spreading layer.

The substrate and the buffering agent of the present invention are contained in layers different from each other. In this case, the component in upper layer laminated is contained in a binder which is soluble in an organic solvent and impervious to water. More preferably, the above binder is water-insoluble. In this case, a solvent which does not dissolve the substrate for ALP or the buffering agent contained in the upper layer is preferred. In this case, the substrate or the buffering agent contained in the upper layer is preferably contained in finely dispersed state.

Examples of the binder meeting the above characteristics may include polyvinyl pyrrolidone, methyl cellulose, ethyl cellulose, copoly(vinylpyrrolidone-vinyl acetate) [commercially available under the trade name of Rubiscol from BASF Co.].

The analytical element of the present invention has a layer containing a buffering agent having a pKa value of about 8.0 and a layer containing a non-self-developable substrate for measuring ALP activity on a support.

The support of the present invention may be a material having a strength enough to have the above respective layers and other layers laminated thereon and hold its integrity, namely having self-supporting property. Such support may be any desired appropriate material, having dimensional stability, preferably transparent, namely radiation transmissive, capable of transmitting electro-magnetic waves with wavelengths

4

of about 200 to 900 nm. Useful support materials may include, for example, polystyrene, polyester [for example, poly(ethyleneterephthalate)], polycarbonate, cellulose ester (for example, cellulose triacetate), etc. The support may have a thickness in the range of from about 50 $\mu$m to about 1 mm, preferably from about 80 $\mu$m to about 300 $\mu$m.

On the surface of the support, a subbing layer can be provided, if desired. For example, subbing layers for increasing adhesive strength with the buffering agent layer laminated on the support or other layers with the support may be employed. Otherwise, water absorbing layer, etc. may be employed, if desired.

On the support, a buffering agent layer is provided. The buffering agent layer of the present invention may be preferably a layer containing a hydrophilic polymer as the binder. Examples of the hydrophilic polymer may include gelatin (e.g., acid-treated gelatin, deionized gelatin, etc.), gelatin derivatives (e.g., phthalated gelatin, hydroxyacrylate-grafted gelatin, etc.), agarose, pullulan, pullulan derivatives, polyacrylamide, polyvinyl alcohol, polyvinyl pyrrolidone, etc.

The buffering agent layer may have a dry film thickness of about 1 $\mu$m to about 100 $\mu$m, preferably about 3 $\mu$m to about 30 $\mu$m.

The analytical element of the present invention can determine the wavelength of the reflected light to be measured according to the spectral absorption characteristics of, for example, an indigo derivative, formed from the substrate, but it is preferably about 580 nm to about 680 nm.

The amount of the respective reagents coated to be used for the analytical element of the present invention can vary broadly depending on the liquid to be analyzed. For example, the above buffering agent having a pKa value of about 8 to 11.5 may be coated in an amount generally of about 6 g/m² or less, preferably about 2 to about 5 g/m², while the ALP substrate about 7 g/m² or less, preferably about 0.3 to about 5 g/m². A phosphoric acid receptor may be used within the element, if desired, and generally in an amount of about 5 g/m² to about 20 g/m², preferably about 0.3 to about 15.0 g/m², and can be used in one layer of the reagent layer, the spreading layer or other layers or all the layers. The phosphoric acid receptor is preferably contained in the layer containing the buffering agent.

Further, as the activator for ALP, there may be included divalent cations, for example, $Mg^{2+}$, $Co^{2+}$, $Mn^{2+}$, $Ca^{2+}$, $Zn^{2+}$, $Sr^{2+}$, $Fe^{2+}$, etc. which are available in the form of salts (for example, sulfate, chloride, acetate, asparate, etc.). The activator is selected from the above divalent cation salts, but preferably the salt of magnesium.

An oxidizing agent may be contained as desired. The content may be generally about 3 g/m², preferably about 0.1 to about 2 g/m².

The analytical element as one embodiment of the present invention comprises a support, and a first reagent layer containing a buffering agent, a second reagent layer containing an ALP substrate and a porous spreading layer successively provided thereon.

Another embodiment comprises a support, and a first reagent layer containing an ALP substrate, a second reagent layer containing a buffering agent and a spreading layer successively provided thereon, while the third embodiment comprises a support, and a reagent layer containing a buffering agent and a porous spreading layer containing an ALP substrate.

The fourth embodiment comprises a support, and a reagent layer containing an ALP substrate and a porous spreading layer containing a buffering agent successively provided thereon.

Still another preferred embodiment comprises a support, and a reagent layer containing a phosphoric acid receptor and a porous spreading layer containing an ALP substrate successively provided thereon.

In the embodiments as described above, the phosphoric acid receptor can be shared with the buffering agent, or when different from the buffering agent, can be contained in the same layer. Further, it can be also contained in a different layer. In this case, it may be contained in the same layer as the layer containing the ALP substrate, or alternatively another new layer may be prepared.

## EXAMPLES

The present invention is described in more detail by referring to the following Examples, by which the present invention is not limited at all.

Example 1

On a transparent poly(ethyleneterephthalate) support applied with subbing treatment with a thickness of about 180 μm, each of the compositions shown in Table 1 was applied.

In tha table, SL is a spreading layer, RL - 1 is a first reagent layer and RL - 2 is a second reagent layer.

Table 1

| Layer | Additives | Analytical element of the present invention | | | | | | Comparative analytical element | |
|---|---|---|---|---|---|---|---|---|---|
| | | (I) | (II) | (III) | (IV) | (V) | (VI) | (I) | (II) |
| SL | Fiber for filter paper starting material (40 to 100 mesh) | 101.7 | 101.7 | 101.7 | 101.7 | 101.7 | 101.7 | 101.8 | 101.8 |
| | Styrene-glycidyl methacrylate copolymer (9:1) | 25.6 | 25.6 | 25.6 | 25.6 | 25.6 | 25.6 | 25.6 | 25.6 |
| | Triton X - 100 (trade name, Rohm & Haas Co.) | 14 | 14 | 14 | 14 | 14 | 14 | 14 | 14 |
| | $MgSO_4 \cdot 7H_2O$ | 0.3 | 0.3 | 0.3 | 0.3 | 0.3 | 0.3 | — | — |
| | 5-Bromo-4-chloro-3-indolyl phosphate toluidine salt | 4.3 | 4.3 | 4.3 | 4.3 | — | — | — | — |
| | p-Nitrophenylphosphate tris salt | — | — | — | — | — | — | 2.4 | 2.4 |
| | 2-Amino-2-methyl-propanol | — | — | 1.5 | — | — | 1.5 | 1.5 | — |
| RL-2 | 5-Bromo-4-chloro-3-indolyl phosphate toluidine salt | — | — | — | 4.3 | — | 4.3 | — | — |
| | Disodium carbonate | — | — | — | — | 4.3 | — | — | — |
| | Gelatin | 21.5 | | 21.5 | — | — | — | 1.08 | — |
| | Bisvinylsulfonylmethyl ether | 0.1 | — | 0.1 | — | — | — | 0.1 | — |
| | Rubiscol VA - 28 (trade name, BASF Co.) | — | — | — | 3.3 | 3.3 | 3.3 | — | — |

Table 1 (Contd)

| Layer | Additives | Analytical element of the present invention | | | | | | Comparative analytical element | |
|---|---|---|---|---|---|---|---|---|---|
| | | (I) | (II) | (III) | (IV) | (V) | (VI) | (I) | (II) |
| RL-1 | 5-Bromo-4-chloro-3-indolyl phosphate toluidine salt | — | — | — | — | 4.3 | — | — | — |
| | Disodium carbonate | 4.3 | — | — | — | — | — | 4.3 | — |
| | 2-Amino-2-methyl-propanol | — | 2.6 | 2.6 | 2.6 | 1.5 | 2.6 | — | 3.5 |
| | $MgSO_4 \cdot 7H_2O$ | | | | 0.3 | | | 0.03 | 0.1 |
| | Gelatin | 20 | —- | 20 | — | 20 | — | 20 | 10.0 |
| | Acrylamide-methylmethacrylate copolymer(9:1) | — | 18 | — | 18 | — | 18 | — | — |
| | Bisvinylsulfonylmethyl ether | — | — | — | — | 0.1 | — | — | — |
| | Rubiscol VA - 28 (trade name, BASF Co.) | 0.1 | 0.1 | 0.1 | 0.1 | 0.1 | 0.1 | 0.1 | 0.1 |

0 269 979

For analytical elements (I) to (VI) of the present invention and the comparative analytical elements (I) and (II) shown in Table 1, fog reflective densities (Fog $D_R$) immediately after drying and after 7 days at 40 °C were measured. The results are shown in Table 2. Measurement was conducted at 610 nm for the analytical elements of the present invention, and at 400 nm for the comparative analytical elements.

## Table 2

| Analytical element | Fog reflective densities (Fog $D_R$) | |
|---|---|---|
| | Immediately after drying | After 7 days |
| Analytical element (I) of this invention | 0.385 | 0.438 |
| "      (II) | 0.401 | 0.411 |
| "      (III) | 0.455 | 0.462 |
| "      (IV) | 0.396 | 0.622 |
| "      (V) | 0.437 | 0.581 |
| "      (VI) | 0.423 | 0.533 |
| Comparative analytical element (I) | 0.533 | 1.032 |
| "      (II) | 0.489 | 0.987 |

Example 2

The above analytical elements (I), (II), (III), (VI) and comparative analytical elements (I) and (II) were cut into square shapes of 1.5 cm, assembled in plastic mounts to provide slides for ALP analysis. Subsequently, each 10 µl of human serum of which ALP activity value was assayed to be 30, 87, 286, 601, 911 and 1150 IU/liter according to the IFCC method was spotted on a porous spreading layer and, while performing incubation at 37 °C under control of evaporation of water content, the analytical film of the present invention was subjected to measurement of reflective densities at 2 min. and 5 min. ( $\Delta D_R$) at 400 nm, and the comparative analytical film at 400 nm. The results are shown in Table 3.

## Table 3

| ALP activities (IU/$l$) | Reflective densities (5 min. − 2 min., $\Delta D_R$) | | | | | |
|---|---|---|---|---|---|---|
| | Analytical element of this invention | | | | Comparative analytical element | |
| | (I) | (II) | (III) | (IV) | (I) | (II) |
| 30 | 0.0112 | 0.0104 | 0.0095 | 0.0083 | 0.0012 | 0.0020 |
| 87 | 0.0327 | 0.0306 | 0.0279 | 0.0257 | 0.0058 | 0.0072 |
| 286 | 0.1080 | 0.1001 | 0.0920 | 0.0908 | 0.0216 | 0.0255 |
| 601 | 0.2256 | 0.2105 | 0.1892 | 0.1881 | 0.0455 | 0.0545 |
| 911 | 0.3334 | 0.3117 | 0.2884 | 0.2845 | 0.0706 | 0.0830 |
| 1150 | 0.4216 | 0.3989 | 0.3700 | 0.3542 | 0.0904 | 0.1050 |

As is also apparent from Table 3, the analytical elements of the present invention have greater sensitivities as compared with comparative analytical elements.

Example 3

On a transparent poly(ethylene terephthalate) support applied with subbing treatment with a thickness of about 180 μm, the following layers were successively laminated.

Reagent layer - I Gelatin 5.4 g/m²
Triton X - 100 (trade name) 0.3 g/m²
2-Ethylaminoethanol 4.8 g/m²
N-(2-hydroxyethyl)ethylenediamine-
N,N,N-triacetic acid 1.3 g/m²
$Zn(C_2 H_3 COO)_2 .2H_2 O$ 0.1 g/m²

Reagent layer - II Gelatin 2.8 g/m²
Triton X - 100 (trade name) 0.1 g/m²
Bisvinylsulfonylmethyl ether 0.1 g/m²

Adhesive layer Rubiscol VA - 28 (trade name) 0.8 g/m²

Porous spreading layer Fiber for filter paper starting
material (40 to 100 mesh) 101.7 g/m²
Styrene-glycidyl methacrylate
copolymer (9 : 1) 25.6 g/m²
Triton X - 100 (trade name) 14.0 g/m²
5-Bromo-4-chloro-3-indolyl phosphate
toluidine salt 4.3 g/m²
Tris(hydroxymethylamino)methane 3.3 g/m²
$MgSO_4 .7H_2 O$ 0.3 g/m²

The analytical element for measuring ALP with the above composition was cut into 1.5 cm squares and assembled in a plastic mount. The analytical element as described above is called the analytical element (VII) of the present invention.

Both of the analytical elements (VII) of the present invention, one immediately after drying and the other after treatment at 40 °C and a relative humidity of 55 % for 7 days, were spotted with 10 $\mu$l of water, subjected to measurement of reflective densities ($D_R$) at 610 nm at 37 °C after 4 minutes, further spotted with similarly with 10 $\mu$l of human sera with ALP activity values of 50, 163 and 564 IU/liter according to the IFCC method, followed by measurement of the difference between the reflective densities at 4 min. and 2 min. ($\Delta D_R$). The results are shown in Table 4.

<u>Table 4</u>

|  | Immediately after drying | 40 °C, 55 % 7 days |
|---|---|---|
| H$_2$O (0 IU/$l$) | 0.2463* | 0.2882* |
| 50 IU/$l$ | 0.0150 | 0.0146 |
| 163 IU/$l$ | 0.0554 | 0.0541 |
| 564 IU/$l$ | 0.1990 | 0.1873 |

\* represents $D_R$ and others $\Delta D_R$.

From the results in Table 4, it can be clearly seen that the analytical element (VII) of the present invention has sufficient performance even after storage at 40 °C

Example 4

On a transparent poly(ethyleneterephthalate) support applied with subbing treatment with a thickness of about 180 $\mu$m, the layers with the following compositions were successively applied.

Reagent layer - I Gelatin                15.0 g/m²
Alkanol XC (trade name, Du'Pont)     0.4 g/m²
5-Bromo-4-chloro-3-indolyl phosphate
toluidine salt                4.8 g/m²
MgSO$_4$ .7H$_2$ O                0.3 g/m²
Bisvinylsulfonylmethyl ether        0.1 g/m²


Reagent layer - II Rubiscol VA - 28 (trade name)     3.8 g/m²
Disodium carbonate            4.3 g/m²
Tris(hydroxymethylamino)methane     3.2 g/m²
Sodium metaperiodate        0.4 g/m²


Porous spreading layer Fiber for filter paper starting
material (40 to 100 mesh)        101.7 g/m²
Styrene-glycidyl methacrylate
copolymer (polymerization ratio 9 : 1)    25.6 g/m²

11

Triton X - 100 (trade name)    14.0 g/m²

For the analytical element (VIII) as described above, each 10 μl of ALP deactivated serum (human) 0 IU/liter, and human sera of 50 IU/liter, 163 IU/liter and 564 IU/liter was spotted on the spreading layer, and the reflective density difference between 2 min. and 4 min. ( $\Delta$ D$_R$) was determined under incubation at 37 °C to give the results as shown in Table 5.

## Table 5

| ALP activity | Analytical element (VIII) of this invention |
|---|---|
| 0 IU/$l$ | − 0.0030 |
| 50 IU/$l$ | 0.0123 |
| 163 IU/$l$ | 0.0469 |
| 564 IU/$l$ | 0.1698 |

Thus, it has also been found that the element containing sodium metaperiodate as the oxidizing agent has similarly good performance.

As described above, according to the analytical element of the present invention, remarkable effects of good storage stability together with high sensitivity can be brought about.

## Claims

1. A multi-layer analytical element for measuring alkaline phosphatase in a liquid sample, comprising a substrate for measuring activity of alkaline phosphatase and a buffering agent having a pKa value of about 8 or higher contained in layers separate from each other, characterized in that said substrate is a phosphoric acid ester of a non-self-developable compound or its salt, and the non-self-developable compound formed by the action of alkaline phosphatase is a compound capable of forming a dye through condensation of two molecules in the presence of an oxidizing agent.

2. The analytical element according to Claim 1, wherein said substrate is a substituted or unsubstituted indolyl phosphate or salts thereof.

3. The analytical element according to Claim 2, wherein said substituted or unsubstituted indolyl phosphate or salts thereof is selected from the group consisting of 3-indolyl phosphate and salts thereof, 5-bromo-3-indolyl phosphate and salts thereof, 5-bromo-4-chloro-3-indolyl phosphate and salts thereof, and 5-bromo-4-iodo-3-indolyl phosphate and salts thereof.

4. The analytical element according to Claim 2, wherein said salts of the substituted or unsubstituted indolyl phosphate are selected from salts of an alkali metal and salts of an organic amine.

5. The analytical element according to Claim 4, wherein said organic amine is selected from the group consisting of toluidine, tris(hydroxymethyl)aminomethane, 2-amino-2-methyl-1,3-propanediol, 2-amino-2-ethyl-1,3-propanediol, bis(cyclohexyl)amine, 2-(ethylamino)ethanol, N-methyl glucamine, 2-(methylamino)-ethanol, 2-(dimethylamino)ethanol, 2-(isopropylamino)ethanol and 2-amino-2-methylpropanol.

6. The analytical element according to Claim 1, wherein said buffering agent is carbonates, borates, Good's buffering agent, diethyanolamine, 2-amino-2-methyl-propanol, tris(hydroxymethyl)aminomethane, diethylamine, 2-(methylamino)ethanol and 2-(isopropylamino)ethanol.

7. The analytical element according to Claim 1, wherein a phosphoric acid receptor is contained in one layer of the reagent layer, the spreading layer or other layers or all the layers.

8. The analytical element according to Claim 1, wherein said analytical element comprises a support, and a first reagent layer containing a buffering agent, a second reagent layer containing an alkaline phosphatase substrate and a porous spreading layer successively provided thereon.

9. The analytical element according to Claim 1, wherein said analytical element comprises a support, and a first reagent layer containing an alkaline phosphatase substrate, a second reagent layer containing a buffering agent and a spreading layer successively provided thereon.

10. The analytical element according to Claim 1, wherein said analytical element comprises a support, and a reagent layer containing a buffering agent and a porous spreading layer containing an alkaline phosphatase substrate.

11. The analytical element according to Claim 1, wherein said analytical element comprises a support, and a reagent layer containing an alkaline phosphatase substrate and a porous spreading layer containing a buffering agent successively provided thereon.

12. The analytical element according to Claim 1, wherein said analytical element comprises a support, and a reagent layer containing a phosphoric acid receptor and a porous spreading layer containing an alkaline phosphatase substrate successively provided thereon.